(19) **Europäisches Patentamt**
**European Patent Office**
**Office européen des brevets**

(11) **EP 2 379 744 B1**

(12) **EUROPEAN PATENT SPECIFICATION**

(45) Date of publication and mention
of the grant of the patent:
**12.10.2016 Bulletin 2016/41**

(51) Int Cl.:
**C12Q 1/68** (2006.01)

(21) Application number: **09799159.0**

(22) Date of filing: **14.12.2009**

(86) International application number:
**PCT/IB2009/055734**

(87) International publication number:
**WO 2010/070572 (24.06.2010 Gazette 2010/25)**

(54) **METHOD FOR THE DETECTION OF DNA METHYLATION PATTERNS**

VERFAHREN ZUM NACHWEIS VON DNA-METHYLIERUNGSMUSTERN

PROCÉDÉ DE DÉTECTION DE PROFILS DE MÉTHYLATION D'ADN

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.12.2008 US 138667 P**

(43) Date of publication of application:
**26.10.2011 Bulletin 2011/43**

(73) Proprietor: **Koninklijke Philips N.V.**
**5656 AE Eindhoven (NL)**

(72) Inventors:
• **DIMITROVA, Nevenka**
**NL-5656 AE Eindhoven (NL)**
• **MITTAL, Chetan**
**NL-5656 AE Eindhoven (NL)**
• **KAMALAKARAN, Sitharthan**
**NL-5656 AE Eindhoven (NL)**

(74) Representative: **Kroeze, Johannes Antonius**
**Philips Intellectual Property & Standards**
**High Tech Campus 5**
**5656 AE Eindhoven (NL)**

(56) References cited:
• **LIDIA LOPEZ-SERRA ET AL: "A Profile of Methyl-CpG Binding Domain Protein Occupancy of Hypermethylated Promoter CpG Islands of Tumor Suppressor Genes in Human Cancer" PROCEEDINGS OF THE AMERICAN ASSOCIATION FOR CANCER RESEARCH ANDAMERICAN SOCIETY OF CLINICAL ONCOLOGY, XX, XX, vol. 66, no. 17, 1 September 2006 (2006-09-01), pages 8342-8346, XP007908119**
• **BALLESTAR E ET AL: "Methyl-CpG binding proteins identify novel sites of epigenetic inactivation in human cancer" EMBO JOURNAL, OXFORD UNIVERSITY PRESS, SURREY, GB, vol. 22, no. 23, 1 December 2003 (2003-12-01), pages 6335-6345, XP002996407 ISSN: 0261-4189**
• **ROBERTSON K.D.: "dna methylation and human disease" NATURE REVIEWS GENETICS, vol. 6, August 2005 (2005-08), pages 597-610, XP002570215**
• **ROBERTSON K.D. & WOLFFE A.: "dna methlyation in health and disease" NATURE REVIEWS GENETICS, vol. 1, October 2000 (2000-10), pages 11-19, XP002570216 cited in the application**

Printed by Jouve, 75001 PARIS (FR)

**Description**

FIELD OF THE INVENTION

The present invention relates to a method for the detection of a DNA methylation signature associated with the presence of or the predisposition to develop a disorder, namely a cancer, the method comprising the identification of one or more candidate genes exhibiting differential DNA methylation in target and reference samples as well as the respective determination of the nucleic acid sites in said candidate genes that are differentially methylated and the recognition sites for DNA binding factors, said DNA binding factors each recognizing such a differentially methylated nucleic acid site, wherein the patterns of differentially methylated nucleic acid sites and of DNA binding factor recognition sites obtained together represent a DNA methylation signature that is indicative for the presence of or the predisposition to develop a cancer in a target sample. In specific applications, these DNA methylation signatures allow for the classification of patient samples, the discrimination between disease subtypes and/or disease states as well as the monitoring of responsiveness to therapy.

BACKGROUND OF THE INVENTION

DNA methylation is found in the genomes of diverse organisms including both prokaryotes and eukaryotes. In prokaryotes, DNA methylation occurs on both cytosine and adenine bases and encompasses part of the host restriction system. In multicellular eukaryotes, however, methylation seems to be confined to cytosine bases and is associated with a repressed chromatin state and inhibition of gene expression (reviewed, for example, in Wilson, G.G. and Murray, N.E. (1991) Annu. Rev. Genet. 25, 585-627).

In mammalian cells, DNA methylation occurs predominantly at CpG dinucleotides, which are distributed unevenly and are underrepresented in the genome. Clusters of usually unmethylated CpGs (referred to as CpG islands) are found in many promoter regions (reviewed, e.g., in Li, E. (2002) Nat. Rev. Genet. 3, 662-673). Changes in DNA methylation leading to aberrant gene silencing have been demonstrated in several human cancers (reviewed, e.g., in Robertson, K.D. and Wolffe, A.P. (2000) Nat. Rev. Genet. 1, 11-19). Hypermethylation of promoters was demonstrated to be a frequent mechanism leading to the inactivation of tumor suppressor genes (Bird, A.P. (2002) Genes Dev. 16, 6-21).

DNA methylation can lead to the silencing of genes by means of two distinct mechanisms: first, methylation at CpG dinucleotide sites that prevents the binding of transcription factors with their cognate DNA recognition sequences; and second, recognition of methyl-CpG dinucleotide sites by a family of methyl-CpG binding proteins (MBD), thus eliciting the repressive potential of methylated DNA.

Various methods exist for experimentally determining differential methylation in individual genes (reviewed, e.g., in Rein, T. et al. (1998) Nucleic Acids Res. 26, 2255-2264). These techniques include *inter alia* bisulfite sequencing, methylation specific PCR (MSP), Methylight and pyro-sequencing. Bisulfite modification converts unmethylated cytosine residues to uridines but the methylated cytosine residues remain unaffected.

Bisulfite sequencing is commonly thought to represent the most appropriate method in order to get an overview of the methylation status of a given genomic sequence. MSP is the most prevalent method in the literature mainly because it enables the analysis of the DNA methylation status in a very limited amount of biological material. Methylight and pyro-sequencing are quantitative PCR based methods. Several genome wide methodological approaches have also been established so far including restriction landmark genomic scanning (RLGS), amplification of inter-methylated sites (AIMS), differential methylation hybridization (DMH), and methylated DNA immunoprecipitation (methyl-DIP).

However, all these methods run out in the physical analysis of the methylation status at a particular site in a gene sequence but do not provide further information on the functional impact of this methylation status with regard to silencing of a particular gene.

Therefore, there remains a need for a method functionally linking the methylation status of a candidate gene with transcriptional gene regulation.

OBJECT AND SUMMARY OF THE INVENTION

It is an objective of the present invention to provide novel approaches for detecting DNA methylation patterns of one or more candidate genes/loci and for functionally correlating these patterns with the transcriptional silencing of said candidate genes.

The DNA methylation signatures thus obtained may be applied as molecular markers in targeted diagnostic tests for screening, diagnosis, prognosis, and recurrence monitoring

In particular, it is an objective of the present invention to provide a method for the detection of a DNA methylation signature associated with the presence of or the predisposition to develop a disorder based on the identification of one or more candidate genes/loci exhibiting differential DNA methylation in target and reference samples as well as the identification of the respective nucleic acid sites in said candidate genes/loci that are differentially methylated and the recognition sites for DNA binding factors, said DNA binding factors each recognizing such a differentially methylated nucleic acid site, wherein the patterns of differentially methylated nucleic acid sites and of DNA binding factor recognition sites obtained together represent a DNA methylation signature that is indicative for the presence of or the predisposition to develop a disorder in a target sample.

It is another specific objective of the present invention to determine the subset of candidate genes/loci present in a given (biological) sample that are hyper-methylated and thus may participate in tumor suppressor gene silencing and the subset of candidate genes that are hypo-methylated and thus may be involved in oncogene activation and to evaluate the potential impact of the respective candidate genes/loci on gene silencing by means of identifying the transcription factors and/or methyl-protein binding proteins recognizing the candidate genes/loci at the sites of differential methylation.

These objectives as well as others, which will become apparent from the ensuing description, are attained by the subject matter of the independent claims. Some of the preferred embodiments are defined by the subject matter of the dependent claims.

In one embodiment, the present invention relates to a method for the detection of a DNA methylation signature associated with the presence of or the predisposition to develop a cancer, the method comprising:

(a) providing a plurality of matched samples, the plurality comprising at least one target sample and at least one reference sample;

(b) identifying one or more candidate genes/loci exhibiting differential DNA methylation in the at least one target sample as compared to the at least one reference sample;

(c) determining the nucleic acid sites comprised in the one or more candidate genes/loci obtained in step (b) that are differentially methylated and subsequently dividing the one or more candidate genes/loci that are differentially methylated in

- a first subset "m" of one or more candidate genes/loci comprising nucleic acid sites which are methylated in the at least one reference sample and unmethylated in the at least one target sample; and
- a second subset "n" of one or more candidate genes/loci comprising nucleic acid sites which are unmethylated in the at least one reference sample and methylated in the at least one target sample; and

(d) determining in the one or more candidate genes/loci obtained in step (b) the presence of recognition sites for DNA binding factors, wherein said DNA binding factors each recognize a nucleic acid site determined in step (c) wherein said step comprises

- determining and selecting the recognition sites for a first subset "M" of one or more DNA binding factors, wherein each member of the subset "M" of DNA binding factors selectively recognizes one or more candidate genes of the subset "m";
- determining and selecting the recognition sites for a second subset "N" of one or more DNA binding factors, wherein each member of the subset "N" of DNA binding factors selectively recognizes one or more candidate genes of the subset "n"; and

  further comprising determining for each member of the subset "M" of DNA binding factors selected the candidate genes/loci comprised in subset "m" that are recognized and/or determining for each member of the subset "N" of DNA binding factors selected the candidate genes/loci comprised in subset "n" that are recognized;

  wherein the pattern of differentially methylated nucleic acid sites obtained in step (c) and the pattern of DNA binding factor recognition sites obtained in step (d) together represent a DNA methylation signature that is indicative for the presence of or the predisposition to develop a cancer in the at least one target sample, wherein

- the nucleic acid sites comprised in the one or more candidate genes/loci that are differentially methylated are CpG dinucleotide sites,
- the DNA methylation signature identified for the subset "m" of candidate genes/loci is indicative of the activation of one or more oncogenes, and
- the DNA methylation signature identified for the subset "n" of candidate genes/loci is indicative of the inactivation of one or more tumor suppressor genes.

Thus, in a method according to the invention, the nucleic acid sites comprised in the one or more candidate genes/loci that are differentially methylated are CpG dinucleotide sites.

Differential DNA methylation is preferably determined by means of one or more methods selected form the group of bisulfite sequencing, pyro-sequencing, methylation-sensitive single-strand conformation analysis (MS-SSCA), high resolution melting analysis (HRM), methylation-sensitive single nucleotide primer extension (MS-SnuPE), base-specific cleavage/MALDI-TOF, methylation-specific PCR (MSP), microarray-based methods, and *Msp*I cleavage.

According to the invention, step (c) of the method comprises dividing the one or more candidate genes/loci that are differentially methylated in

- a first subset "m" of one or more candidate genes/loci comprising nucleic acid sites which are methylated in the at least one reference sample and unmethylated in the at least one target sample; and
- a second subset "n" of one or more candidate genes/loci comprising nucleic acid sites which are unmethylated in the at least one reference sample and methylated in the at least one target sample.

In addition, step (d) of the method comprises determining and selecting the recognition sites for a first subset "M" of one or more DNA binding factors, wherein each member of the subset "M" of DNA binding factors selectively recognizes one or more candidate genes of the subset "m".

Furthermore, step (d) of the method comprises determining and selecting the recognition sites for a second subset "N" of one or more DNA binding factors, wherein each member of the subset "N" of DNA binding factors selectively recognizes one or more candidate genes of the subset "n".

Particularly preferably, the subset "N" of DNA binding factors represents DNA methyl-binding proteins. In further specific embodiments, the DNA methyl-binding proteins are selected from the group of MBD1, MBD2, MBD3, MBD4, MIZF, Kaiso, and MeCP2.

The method of the invention further comprises determining for each member of the subset "M" of DNA binding factors selected the candidate genes/loci comprised in subset "m" that are recognized and/or determining for each member of the subset "N" of DNA binding factors selected the candidate genes/loci comprised in subset "n" that are recognized.

In yet another specific embodiment, the method of the invention further comprises one or more repetitions of step (d), wherein each repetition comprises determining in the one or more candidate genes/loci the presence of recognition sites for one or more DNA binding factors that have not been included in the determination of the previous repetition(s).

Preferably, the DNA methylation signature identified comprises at least ten candidate genes.

According to the invention, the DNA methylation signature of the one or more candidate genes identified is indicative for the presence of or the predisposition to develop a cancer in the at least one target sample. In particular, the DNA methylation signature identified for the subset "m" of candidate genes is indicative of the activation of one or more oncogenes and the DNA methylation signature identified for the subset "n" of candidate genes is indicative of the inactivation of one or more tumor suppressor genes.

In further specific embodiments, the method is performed *in silico.*

In a further specific embodiment, the method of the invention is for the further use of predicting the therapeutic response to the treatment of the disorder present or predisposed to develop in the at least one target sample.

In another embodiment, the present invention relates to the use of a DNA methylation signature as defined herein as a biomarker for the classification of patient samples for screening, diagnosing, therapy planning and/or recurrence monitoring of a disorder.

In yet another embodiment, the present invention relates to the use of the method as an integral part of a computer-based clinical decision system along with other patient data and clinical parameters.

DESCRIPTION OF THE FIGURES

Figure 1    depicts a schematic illustration of an exemplary method for the determination of differentially methylated nucleic acid sites comprised in a candidate gene that is based on methylation-dependent restriction analysis. The principle of the method is outlined in more detail in the experimental section.

Figure 2    shows an exemplary distribution of clustered samples (columns) vs. methylation loci (rows). The DNA methylation patterns obtained allow for a differentiation between tumors (left part of bar on top) and normal tissue (right part of bar on top).

Figure 3    represents a schematic illustration of the general principle of the method according to the invention.

Figure 4    (A) shows a list of differentially methylated loci (identified using *Msp*I cleavage and a MOMA array) which were found indicative for distinguishing between different subtypes of breast cancer, namely luminal A *vs.* basal and Her2. (B) depicts a table including the type of methyl-binding protein (MBP), the *Msp*I fragment (MSP) in which a MBP binding site was identified, the distance to the closest gene, and the name of said closest gene.

DETAILED DESCRIPTION OF THE INVENTION

The present invention is based on the unexpected finding that the determination of a DNA methylation signature based on the pattern of differentially methylated nucleic acid sites comprised in one or more candidate genes/loci and the pattern of recognition sites for DNA binding factors recognizing said nucleic acid sites of differential methylation enables the reliably detection of the presence of or the predisposition to develop a disorder in a given sample.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are to be considered non-limiting.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun e.g. "a" or "an", "the", this includes a plural of that noun unless specifically stated otherwise.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of terms will be given in the context of which the terms are used. The following terms or definitions are provided solely to aid in the understanding of the invention and should not be construed to have a scope less than understood by a skilled person.

In a first aspect, the present invention relates to a method for the detection of a DNA methylation signature associated with the presence of or the predisposition to develop a cancer, the method comprising:

(a) providing a plurality of matched samples, the plurality comprising at least one target sample and at least one reference sample;

(b) identifying one or more candidate genes/loci exhibiting differential DNA methylation in the at least one target sample as compared to the at least one reference sample;

(c) determining the nucleic acid sites comprised in the one or more candidate genes/ loci obtained in step (b) that are differentially methylated and subsequently dividing the one or more candidate genes/loci that are differentially methylated in

- a first subset "m" of one or more candidate genes/loci comprising nucleic acid sites which are methylated in the at least one reference sample and unmethylated in the at least one target sample; and
- a second subset "n" of one or more candidate genes/loci comprising nucleic acid sites which are unmethylated in the at least one reference sample and methylated in the at least one target sample; and

(d) determining in the one or more candidate genes/loci obtained in step (b) the presence of recognition sites for DNA binding factors, wherein said DNA binding factors each recognize a nucleic acid site determined in step (c), wherein said step comprises

- determining and selecting the recognition sites for a first subset "M" of one or more DNA binding factors, wherein each member of the subset "M" of DNA binding factors selectively recognizes one or more candidate genes of the subset "m";
- determining and selecting the recognition sites for a second subset "N" of one or more DNA binding factors, wherein each member of the subset "N" of DNA binding factors selectively recognizes one or more candidate genes of the subset "n"; and

further comprising determining for each member of the subset "M" of DNA binding factors selected the candidate genes/loci comprised in subset "m" that are recognized and/or determining for each member of the subset "N" of DNA binding factors selected the candidate genes/loci comprised in subset "n" that are recognized; wherein the pattern of differentially methylated nucleic acid sites obtained in step (c) and the pattern of DNA binding factor recognition sites obtained in step (d) together represent a DNA methylation signature that is indicative for the presence of or the predisposition to develop a cancer in the at least one target sample, wherein

- the nucleic acid sites comprised in the one or more candidate genes/loci that are differentially methylated are CpG dinucleotide sites,

- the DNA methylation signature identified for the subset "m" of candidate genes/loci is indicative of the activation of one or more oncogenes, and
- the DNA methylation signature identified for the subset "n" of candidate genes/loci is indicative of the inactivation of one or more tumor suppressor genes.

The respective target samples and reference samples used in the present invention may be derived of prokaryotic or eukaryotic origin. Typically, the samples employed are mammalian samples that may be of human or non-human origin, with human samples being preferred. The term "sample", as used herein, is to be understood not only to include individual cells but also tissues, organs, and organisms.

The term "target sample", as used herein, refers to a sample being at least supposed to exhibit or to have a predisposition to develop a disorder, whereas the term "reference sample" (also referred to as "control sample") typically denotes wild-type material (e.g., healthy cells) not having characteristics of such a disorder. However, in some applications, the method of the invention may be used to analyze and compare several samples exhibiting characteristics of a disorder (e.g., pre-disease and disease states), for example in order to monitor disease progression. In such scenario, if no wild-type (healthy) control sample is included, the sample having the less severe disease characteristics is typically considered the "reference sample".

The term "matched samples", as used herein, denotes a plurality of at least two samples that relate to each other. For example, a pair of samples to be analyzed may include one target sample derived from a patient suffering from a disease (e.g., cancer) and one reference sample derived from a healthy subject. However, the method of the invention is not restricted to the analysis of pairs of samples. For example, it is also possible to analyze four different target samples derived in comparison to one reference sample, e.g., target samples from patients suffering from the same disease but being affected to different extents (e.g., three different pre-cancerous states and one cancer sample).

Accordingly, the term "plurality of matched samples", as used herein, denotes any even or odd number of samples that is $\geq 2$ (for example, 2, 3, 4, 5, 6, 7, 8, 9, 10, 11, 12 and so forth) as long as the plurality of samples encompasses at least one target sample and at least one reference sample.

Typically, the target and reference samples used in the present invention are derived from biological materials collected from the subjects to be treated. Furthermore, in order to corroborate the data obtained "comparative samples" may also be collected from subjects having a given known disease state. The biological samples may include body tissues (e.g., biopsies or resections) and/or body fluids, such as blood, sputum, and urine. Furthermore, the biological samples may contain cell extracts derived from or a cell population of a subject.

Optionally, the cells or cell extracts may be purified from the obtained body tissues and fluids, if necessary, and then used as the biological sample. The samples used in the methods of the present invention should generally be collected in a clinically acceptable manner, preferably in a way that nucleic acids or proteins are preserved.

The term "disorder", as used herein, is to be understood in the broadest sense. The term denotes: (i) any type of medical condition, that is, any morphological and/or physiological alterations in the target samples (i.e. the cells and/or tissues) exhibiting characteristics of a dysfunctional and/or aberrant cellular phenotype a as compared to unaffected (wild-type) control samples; and/or (ii) any morphological, physiological and/or pharmacological difference between the respective target and reference samples. Examples of alterations according to (i) may relate *inter alia* to cell size and shape (enlargement or reduction), cell proliferation (increase in cell number), cell differentiation (change in physiological state), apoptosis (programmed cell death) or cell survival. Examples of differences according to (ii) includes *inter alia* tumor samples *vs.* healthy controls (for the purpose of diagnosis or recurrence monitoring), aggressive *vs.* non-aggressive tumor samples (i.e. different tumor stages and/or tumor sub-types; for the purpose of prognostic analyses), conditions relating to treatment regimens such as responsiveness *vs.* non-responsiveness to a particular therapy for a given disorder/medical condition. Thus, the term disorder may be interpreted as a any kind of difference between two or more samples based on which said samples may be distinguished and/or classified.

In a method according to the present invention, the disorder is a cancer, that is, a type of malignant neoplasm (also referred to as carcinoma) including *inter alia* colon, lung, liver, breast, ovary, and pancreas cancer, melanoma, neuronal tumors (e.g., gliobastoma, astrocytoma, medullobastoma), and the like.

The term "having a predisposition to develop a disorder", as used herein, denotes any cellular phenotype being indicative for a pre-state of a disorder, i.e. an intermediate state in the transformation of a normal into an aberrant phenotype. In other words, the term denotes a state of risk of developing a disorder.

The term "identifying one or more candidate genes/loci", as used herein, should be interpreted in the sense of "selecting" at least one candidate gene from the group of genes present in a given sample that undergo differential methylation. The term "candidate genes" (herein also referred to as "candidate loci"), as used herein, relates to any genetic loci comprising in their nucleic acid sequence one or more nucleic acid sites that may be present in a methylated state and in an unmethylated state. Within the context of the present invention, the term gene is not necessarily restricted to sequences (open reading frames) encoding a protein but includes intergenic regions as well. The selection (i.e. the number and/or type of candidate genes/loci chosen) may vary, for example, depending on treatment modalities of a disease or disorder to be analyzed, including therapeutic intervention, diagnostic criteria such as disease stages, and disease monitoring and surveillance for the disease in the subject to be treated, from whom the sample to be analyzed is derived. Additionally, the term "identifying" encompasses the determination of the extent of the differential DNA methylation in the at least one target sample and the at least one reference sample and comparing the results obtained.

The one or more candidate genes/loci identified may be subjected to further analysis individually or they may be clustered to one or more candidate gene/loci signatures, wherein the entities of each signature are analyzed *en bloc* (i.e. together). The term "candidate gene/loci signatures", as used herein, denotes subsets of at least two candidate genes/loci that are related to each other, for example, encoding functionally equivalent proteins or proteins participating in the same signaling pathway, or the like.

The term "DNA methylation", as used herein, denotes the type of chemical modification of DNA that involves the addition of a methyl group to DNA, for example to the C5 carbon atom of the cytosine pyrimidine ring or to the N6 nitrogen atom of the adenosine purine ring, with the first option being particularly preferred herein. This modification can be inherited and subsequently removed without changing the original DNA sequence. As such, it is part of the epigenetic code and the most well characterized epigenetic mechanism.

DNA methylation at the C5 of cytosine has been found in every vertebrate examined. In adult somatic tissues, DNA methylation typically occurs in a CpG dinucleotide context (cf. below). Non-CpG methylation is prevalent, for example, in embryonic stem cells. DNA methylation is reversible: DNA methyl-transferases catalyze the transfer of a methyl group from S-adenosyl-L-methionine to cytosine or adenosine residues. DNA polymerases do not copy the methylated status during replication (reviewed, e.g., in Robertson, K.D. and Wolffe, A.P. (2000), *supra*; Li, E. (2002), *supra*; Bird, A.P. (2002), *supra*).

The term "differential DNA methylation", as used herein, denotes a condition in which a particular candidate gene is (at one or more nucleic acid sites comprised in its sequence) methylated in the at least one target sample but unmethylated in the at least reference sample, or *vice versa*, in which a particular candidate gene is (at one or more nucleic acid sites comprised in its sequence) unmethylated in the at least one reference sample but methylated in the at least target sample

Generally, the determination of the differential DNA methylation pattern of the one or more candidate genes/loci may be accomplished by any means known in the art. In preferred embodiments, differential DNA methylation is determined by means of one or more methods selected form the group of bisulfite sequencing, pyro-sequencing, methylation-sensitive single-strand conformation analysis (MS-SSCA), high resolution melting analysis (HRM), methylation-sensitive single nucleotide primer extension (MS-SnuPE), base-specific cleavage/MALDI-TOF, methylation-specific PCR (MSP), microarray-based methods, and *Msp*I cleavage (reviewed, e.g., in Rein, T. et al. (1998), *supra*). Further suitable methods are disclosed, e.g., in US patent application 2006/0292564 A1.

The determination of the differential DNA methylation pattern of the one or more candidate genes/loci already includes the identification of the exact nucleic acid sites (i.e. sequence elements, genetic loci) comprised in the one or more candidate genes as defined in step (c) according to the method of the present invention.

In the method according to the invention, the nucleic acid sites comprised in the one or more candidate genes/loci that are differentially methylated are CpG dinucleotide sites.

The term "CpG dinucleotide sites" (or "CpG sites), as used herein, refers to regions of DNA where a cytosine nucleotide is located immediately adjacent to a guanine nucleotide in the linear sequence. "CpG" stands for cytosine and guanine separated by a phosphate (i.e., -C-phosphate-G-). The "CpG" notation is used to distinguish a cytosine followed by guanine from a cytosine base paired to a guanine. There are regions of the DNA that have a higher concentration of CpG sites, known as CpG islands. Many genes in mammalian genomes have CpG islands associated with the transcriptional start site (including the promoter) of the gene.

Hypermethylation (i.e. an increased level of methylation) of CpG sites within the promoters of genes can lead to their silencing, a feature found, e.g., in a number of human cancers (for example the silencing of tumor suppressor genes). In contrast, the hypomethylation (i.e. a reduced level of methylation) of CpG sites has been associated with the over-expression of oncogenes within cancer cells (reviewed, e.g., in Robertson, K.D. and Wolffe, A.P. (2000), *supra*; Li, E. (2002), *supra*; Bird, A.P. (2002), *supra*; Klose, R.J. and Bird, A.P. (2006) Trends Biochem. Sci. 31, 89-97).

According to the present invention, step (c) of the method comprises dividing the one or more candidate genes/loci that are differentially methylated in

- a first subset "m" of one or more candidate genes/loci comprising nucleic acid sites which are methylated in the at least one reference sample and unmethylated in the at least one target sample; and
- a second subset "n" of one or more candidate genes/loci comprising nucleic acid sites which are unmethylated in the at least one reference sample and methylated in the at least one target sample.

Within the context of the present invention, a candidate gene/locus may comprise only a single nucleic acid site that is differentially methylated between the at least one target sample and the at least one reference sample. However, it may also be possible that a particular candidate gene/locus that has more than one such nucleic acid sites, which can be of the same type (i.e. all of them belonging to subset "m" or all of them belonging to subset "n" as defined above) or of different types (i.e. at least one of them belonging to subset "m" and least one other belonging to subset "n" as defined above). In a scenario of more than one differentially methylated nucleic acid site comprised in a particular candidate gene/locus the subsequent analysis of the DNA binding factor recognition sites, as defined in step (d) of the method according to the invention, may be performed separately for each individual nucleic acid site or for the candidate gene in its entirety. Accordingly, the terms "candidate gene/locus" and "nucleic acid" may be used interchangeably herein depending on the type of analysis performed. Thus, the method of the present invention may comprise the selection and analysis of one or more candidate genes, one or more nucleic acid sites or a combination thereof.

In analogy, the DNA methylation state (or level) may refer to an individual nucleic acid site or to the overall methylation level of a candidate gene/locus comprising more than one nucleic acid sites. The latter case appears uncritical if the plurality of nucleic acid sites is of the same type (cf. above). However, in case, the plurality of nucleic acid sites comprises entities of different types a candidate gene/locus is considered to be in the "methylated" state if in the at least one target sample as compared to the at least one reference sample a higher number of unmethylated nucleic acid sites becomes methylated than *vice versa*. One the other hand, a candidate gene/locus is considered to be in the "unmethylated" state if in the at least one target sample as compared to the at least one reference sample a higher number of methylated nucleic acid sites becomes unmethylated than *vice versa*.

The determination of the presence of recognition sites for DNA binding factors in the one or more candidate genes/loci, as defined in step (d) of the method of the invention, may also be accomplished by any means known in the art. Typically, this goal is accomplished by comparing the consensus sequences for the respective DNA recognition (i.e. binding) sites of one or more DNA binding factors (available from the literature or from databases such as TRANSFAC; Wingender, E. et al. (1996) Nucleic Acids Res. 24, 21-25) with the nucleic acid sequences of the one or more candidate genes to be analyzed. Within the present invention, only those recognition sites are considered that are identical or overlap with a site of differential methylation in such way that recognition/binding of a DNA binding factor to a recognition site as defined herein prevents methylation of this site or, *vice versa,* methylation of the site prevents recognition/binding of a DNA binding factor to the site. In other words, there is a mutual exclusivity of DNA methylation and DNA binding factor recognition at a given nucleic acid site comprised in a candidate gene.

The term "DNA binding factor", as used herein, denotes any protein binding to a specific sequence element in a target DNA molecule thus exerting any effects with regard to the expression of said DNA molecule, preferably at the transcriptional level, that is, either an activation (or enhancement) or a repression (or silencing) of gene expression. Hence, DNA binding factors are commonly also referred to as "transcription factors". Examples of such DNA binding factors include *inter alia* the eukaryotic general transcription factors (TFIIA, TFIIB, TFIID TFIIE, TFIIF, and TFIIH) involved in assembly of the transcriptional pre initiation complex, upstream transcription factors (binding somewhere upstream of the initiation site to stimulate or repress transcription), and inducible transcription factors (being similar to upstream transcription factors but requiring activation or inhibition). Specific examples for the latter two classes include, e.g., helix-loop-helix/leucine zipper factors, zinc finger factors, helix-turn-helix factors (e.g., homeo domain factors, fork head/winged helix factors, heat shock factors), beta-scaffold factors (e.g., STAT factors, TATA binding factors) as well as methyl-DNA binding proteins, with the latter ones being particularly preferred (cf. below).

As outlined above, DNA methylation can lead to the silencing of genes by means of two distinct mechanisms: first, methylation at CpG dinucleotide sites that prevents the binding of transcription factors with their cognate DNA recognition sequences to such sites; and second, recognition of methyl-CpG dinucleotide sites by a family of methyl-CpG binding proteins (MBD), thus eliciting the repressive potential of methylated DNA. In the first-mentioned scenario, the recognition sequences resemble the first subset "m" of candidate genes/differentially methylated nucleic acid sites which are methylated in the at least one reference sample and unmethylated in the at least one target sample. In the second scenario, the recognition sequences resemble the second subset "n" of candidate genes/differentially methylated nucleic acid sites which are unmethylated in the at least one reference sample and methylated in the at least one target sample. Accordingly, different subsets of DNA binding factors and DNA binding factor recognition sites may be defined, respectively.

According to the invention, step (d) of the method further comprises determining and selecting the recognition sites for a first subset "M" of one or more DNA binding factors, wherein each member of the subset "M" of DNA binding factors selectively recognizes one or more candidate genes of the subset "m".

In addition, step (d) of the method further comprises determining and selecting the recognition sites for a second subset "N" of one or more DNA binding factors, wherein each member of the subset "N" of DNA binding factors selectively recognizes one or more candidate genes of the subset "n".

In particularly preferred embodiments of the invention, the subset "N" of DNA binding factors represents DNA methyl-binding proteins. Most preferably, the DNA methyl-binding proteins are selected from the group of MBD1, MBD2, MBD3, MBD4, MIZF, Kaiso, and MeCP22.

The term "DNA methyl-binding proteins", as used herein, denotes a specific family of DNA binding factors which specifically recognize methylated DNA sequences, particularly methylated CpG dinucleotides (mCpG). DNA methyl-binding proteins are were identified more than a decade ago (reviewed, e.g., in Bird, A.P. and Wolffe, A.P. (1999) Cell 99, 451-454; Wade, P.A. (2001) BioEssays 23, 1131-1137; Hendrich, B. and Tweedie, S. (2003) Trends Genet. 19, 269-277). Characterization of the methyl-CpG-binding domain (MBD) - the protein motif responsible for binding to methylated CpG dinucleotides - facilitated bioinformatic identification of a family of proteins that share this domain. With the exception of MBD3, which contains amino acid substitutions that prevent binding to methyl-CpG, the mammalian MBD proteins (named MBD1-MBD4) and the founding member, MeCP2, all specifically recognize methyl-CpG. MIZF denotes the MBD2-interacting zinc finger which constitutes a component of the MeCP1 histone deacetylase (HDAC) complex. A novel MBP named Kaiso lacks the MBD, but recognizes methylated DNA through zinc-finger domains. All MBPs can mediate silencing of gene expression. This is accomplished by targeting chromatin remodelling co-repressor complexes to regions containing DNA methylation. All six methyl DNA binding proteins specifically mentioned herein are well known in the art. Their nucleic acid sequences as well as their DNA recognition consensus motifs can be derived from databases such as GeneBank.

The nucleotide sequences of the corresponding six human genes are deposited in GenBank having the following accession numbers:

MBD1:NM_015846 (isoform 1; in total, four isoforms)
MBD2:NM_003927 (isoform 1; in total, two isoforms)
MBD3:NM_003926
MBD4:NM_003925
MIZF: NM_015517
MeCP2: NM_004992 (isoform 1; in total, two isoforms)
Kaiso: NM_006777

According to the present invention, the above analysis for determining the presence of recognition sites for DNA binding factors may be performed for an individual nucleic acid site comprised in a candidate gene/locus that is differentially methylated, for two or more such sites comprised in an individual candidate gene/locus, and for two or more candidate genes/loci, each gene comprising one or more differentially methylated sites, respectively, wherein two or more sites may be analyzed concomitantly or sequentially. Furthermore, any of these analyses may be performed with regard to the recognition site(s) for one or more DNA binding factor(s), wherein the evaluation of the presence of consensus binding sequences may gain be performed either concomitantly or sequentially.

In some embodiments, the method of the invention further comprises in step (d) one or more cycles of analysis, each cycle comprising the determination of the DNA binding factor recognition sites in one or more candidate genes identified and the subsequent selection of one or more subgroups of recognition sites for DNA binding factors that are present in the one or more candidate genes/loci or, *vice versa*, that are lacking in the one or more candidate genes/loci. Only the selected one or more subgroups (and thus only the candidate gene(s)/loci comprising the respective binding sites) are then subjected to another cycle of analysis. Thus, the method of the invention may comprise one or more repetitions of step (d), wherein each cycle of repetition comprises determining in the one or more candidate genes/loci the presence of recognition sites for one or more DNA binding factors that have not been included in the determination of the previous repetitions performed.

For example, in a first round of analysis the subset N of DNA binding factors, as defined above, is selected. Thus, only those candidate genes comprising binding sites for this subset N of DNA binding factors (i.e. candidate genes of the subset "n") will be further considered. It is then assumed that the subset N of DNA binding factors comprises one or more (structurally and/or functionally related) families of transcription factors designated subgroups N1, N2, N3, N4, N5, N6, and so forth. In the second round, only the subgroup N1 (for example, methyl DNA binding proteins) should be analyzed. Thus, only a corresponding subgroup "n1" of candidate genes comprising recognition sites for N1 will be selected and subjected to a third round of analysis. Now, it is assumed that subgroup N1 encompasses multiple individual DNA binding factors designated $N1_1$, $N1_2$, $N1_3$, $N1_4$, $N1_5$, $N1_6$, and so forth. In the third round, only $N1_1$ and $N1_2$ (for example, MBD1 and MBD2) should be analyzed. Hence, after three selection cycles only those candidate genes remain whose expression is influenced by binding of $N1_1$ and $N1_2$.

It is possible as well to combine in an analysis positive (presence of a specific recognition site) and negative features (absence of another specific recognition site). Accordingly, by employing this approach genetic networks can be elucidated, for example candidate genes whose methylation-dependent expression is regulated by a single transcription factor.

The method of the invention further comprises determining for each member of the subset "M" of DNA binding factors selected the candidate genes comprised in subset "m" that are recognized and/or determining for each member of the subset "N" of DNA binding factors selected the candidate genes comprised in subset "n" that are recognized.

Thus, the method of the present invention results in the determination of the pattern of differentially methylated nucleic acid sites comprised in one or more candidate genes/loci and in the determination of the pattern of recognition sites for DNA binding factors specifically binding to differentially methylated nucleic acid sites. Correlating these patterns adds up to a unique DNA methylation signature that is indicative for the presence of or the predisposition to develop a disorder in the at least one target sample

The term "DNA methylation signature" (also referred to as "biosignature"), as used herein, denotes a set of one or more candidate genes/loci having a particular pattern of DNA methylation and a corresponding particular pattern of DNA binding factor recognition sites present in their nucleic acid sequence. This unique combination allows for identifying a phenotypic state (e.g., a disorder) in a target sample that enables distinguishing the target sample from a reference sample.

In other words, according to the present invention the DNA methylation signature in its entirety (i.e. the one or more differentially methylated candidate genes together) is indicative for the presence of a disorder but not the mere differential methylation of any individual candidate gene(s)/loci as such. Within the context of the present invention, the determination of the pattern of DNA binding factor recognition sites may also be considered as a "filtering system" for increasing the significance of a DNA methylation signature/biosignature for a particular application (for example, for diagnosing a specific tumor subtype and for distinguishing said subtype from other probably closely related subtypes). Thus, such a filter may be used for prioritizing the candidate genes/loci included in such a biosignature based on their regulatory potential (i.e. based on the conservation value of the sequence and the presence of regulatory elements therein).

In specific embodiments, the DNA methylation signature comprises at least three or at least five candidate gene. Preferably, the DNA methylation signature comprises at least 10 candidate genes (e.g., 12, 15, 20, 50, 100, 200, 1000 or more).

In further specific embodiments, the method is performed *in silico.* The term "*in silico*", as used herein, is to be understood as "performed on a computer or via computer simulation." The method of the invention may be completely performed via computer simulation or at least in part, that is, by combining experimental approaches with computer simulations. This may depend on the particular application, the type of samples to be analyzed, the condition to be detected or diagnosed, and the like. Methods and software for performing such computer simulations are commercially available and well known in the art.

In a method according to the invention, the DNA methylation signature of the one or more candidate genes/loci identified is indicative for the presence of or the predisposition to develop a cancer in the at least one target sample (cf. also the discussion above). In particular, the DNA methylation signature identified for the subset "m" of candidate genes/loci is indicative of the activation of one or more oncogenes and the DNA methylation signature identified for the subset "n" of candidate genes/loci is indicative of the inactivation of one or more tumor suppressor genes.

In a further specific embodiment, the method of the invention is for the further use of predicting the therapeutic response to the treatment of the disorder present or predisposed to develop in the at least one target sample.

In another aspect, the present invention relates to the use of a DNA methylation signature as defined herein as a biomarker for the classification of patient samples for screening, diagnosing, therapy planning and/or recurrence monitoring of a disorder.

In yet another aspect, the present invention relates to the use of the method as an integral part of a computer-based clinical decision system along with other patient data and clinical parameters.

The invention is further described by the figures and the following examples, which are solely for the purpose of illustrating specific embodiments of this invention, and are not to be construed as limiting the scope of the invention in any way.

EXAMPLES

## 1. Samples

Patient samples were obtained from Norwegian Radium Hospital, Oslo, Norway. Patient consent obtained as per legal requirements.

## 2. CpG islands

Annotated CpG islands were obtained from the UCSC genome browser. These islands were predicted using the Gardiner-Garden definition (Gardiner-Garden, M. and Frommer, M. (1987) J. Mol. Biol. 196, 261-82) involving the following criteria: length ≥ 200 bp; % GC ≥ 50%; observed/expected CpG ≥ 0.6. There are ~26219 CpG islands in the size range of 200 bp to 2000 bp in the genome. These islands are well covered by *Msp*I restriction fragmentation.

Arrays were manufactured by Roche NimbleGen Inc. (Madison, WI, USA) using the 390K format to the following specifications. The CpG island annotation from human genome build 33 (hg17) was used to design a 50mer tiling array.

The 50mers were shifted on either side of the island sequence coordinates to evenly distribute the island. The 390K format has 367658 available features which would not fit all islands with a 50mer tiling. Therefore, a cutoff on the islands to be represented based on size was made, with only CpG islands having a size of 200 bp to 2000 bp being assayed. Control probes were designed to represent background signal. Sample preparation was performed essentially as described previously (Lucito, R. et al. (2003) Genome Res. 13, 2291-2305), except the following modifications: (i) The primary restriction endonuclease employed was MspI. (ii) After digestion the linkers MspI24mer, and MSPI12mer were ligated. The 12 mer is not phosphorylated and does not ligate. (iii) After ligation, the material is purified by phenol/chloroform-extraction, precipitated, centrifuged, and re-suspended.

The material is then divided in two aliquots, one half being digested using the restriction endonuclease *Mcr*BC, and the other half being mock digested. Four 250 $\mu$l tubes were used for each sample pair for PCR amplification of the representation each with a 100 $\mu$l volume reaction. The cycle conditions were 95° C for 1 min, 72° C for 3 min for 15 cycles, followed by a 10 min extension at 72° C. Afterwards, the contents of the tubes for each pair were pooled. The samples were cleaned via phenol/chloroform extraction, precipitated, resuspended, and the DNA concentration was determined.

The DNA was labeled as described (Lucito, R. et al. (2003), *supra*) except minor changes. Briefly, 2 $\mu$g DNA template (dissolved in TE buffer, pH 8.0) were placed in a 0.2 ml PCR tube. 5 $\mu$l random nonamers (Sigma-Aldrich Co., St. Louis, MI, USA) were added, the final volume was adjusted to 25 $\mu$l with dH$_2$O, and the sample was mixed. The tubes were incubated at 100° C for 5 min, then on ice for 5 min. To each sample, 5 $\mu$l NEB Buffer 2 (New England Biolabs, Ipswich, MA, USA), 5 $\mu$l dNTPs (0.6 nM dCTP, 1.2 nM each of dATP, dTTP, dGTP), 5 $\mu$l label (Cy3-dCTP or Cy5-dCTP; GE Healthcare Bio-Sciences Corp., Piscataway, NJ, USA), 2 $\mu$l NEB Klenow fragment, and 2 $\mu$l dH$_2$O were added. Procedures for hybridization and washing were essentially as described (Lucito, R. et al. (2003), *supra*) with the exception that oven temperature for hybridization was increased to 50° C. Arrays were scanned using the GenePix 4000B microarray scanner (Molecular Devices, Inc., Sunnyvale, CA, USA) at a pixel size of 5$\mu$m. The GenePix Pro 4.0 software was used to quantify the intensity for the arrays. Array data were imported into S-PLUS statistics software for further analysis.

### 3. Data analysis

Microarray images were scanned on the GenePix 4000B microarray scanner and data extracted using Nimblescan software (Roche NimbleGen Inc., Madison, WI, USA). For each probe, the geometric mean of the ratios (i.e. the GeoMeanRatio) of McrBc and control treated samples were calculated for each experiment and its associated dye swap.

The respective GeoMeanRatios of all the samples in a dataset were then normalized using quantile normalization method (Bolstad, B.M. et al. (2003) Bioinformatics 19, 185-193). The normalized ratios for each experiment were then combined to get one value for all probes in every MspI fragment using a median polish model. The data thus obtainedwere then used for further analysis.

Analysis of variance was used to identify the most significant islands. In order to determine the most consistently occurring changes in methylation between tumor and normal samples, we used a t-test approach. Using a p-value cutoff of 0.001 after correction for multiple testing (False Discovery Rate; Benjamini, Y. and Hochberg, Y. (1995) J. Roy. Stat. Soc., Ser. B 57, 289-300), a list of 916 differentially methylated *Msp*I fragments was obtained.

Supervised learning: A supervised machine learning classifier was employed to identify the number of features required to differentiate tumor samples from normal. A publicly available support vector machine (SVM) library (LibSVM Ver 2.8) was used to obtain classification accuracy using a leave one out method (Lin, C.J. (2001) Neural Computation 13, 307-317) The methylation features for classification were first selected using t-test among the training data alone. The SVM was then trained on the top 10, 50 and 100 features using the radial basis function (RBF) kernel.

For N samples, t-tests were performed for (N-1) samples to identify fragments with significant differences in methylation ratios. For N samples, this analysis was performed N times, omitting each sample once during the t-test calculations. The methylation ratios of top 10 fragment features from (N-1) samples were then used for training the SVM. The ratio from one untrained sample was used as a control. Based on as few as 10 features, a classification accuracy of 94% was accomplished. Interestingly, two samples from timorous tissue that were classified as normal in this analysis were also closest to normal in both gene expression and ROMA analysis, respectively.

### 4. Detection of methylated sites

In one embodiment, the method comprises the isolation of the genomic DNA from the samples such as cell lines, tissue or blood samples. DNA extraction may be accomplished by means that are standard to one skilled in the art including the use of detergent lysates, sonication and vortexing with glass beads. Once the nucleic acids have been extracted, the genomic double stranded DNA is used in the analysis. Optionally, the DNA may be cleaved prior to further analysis.

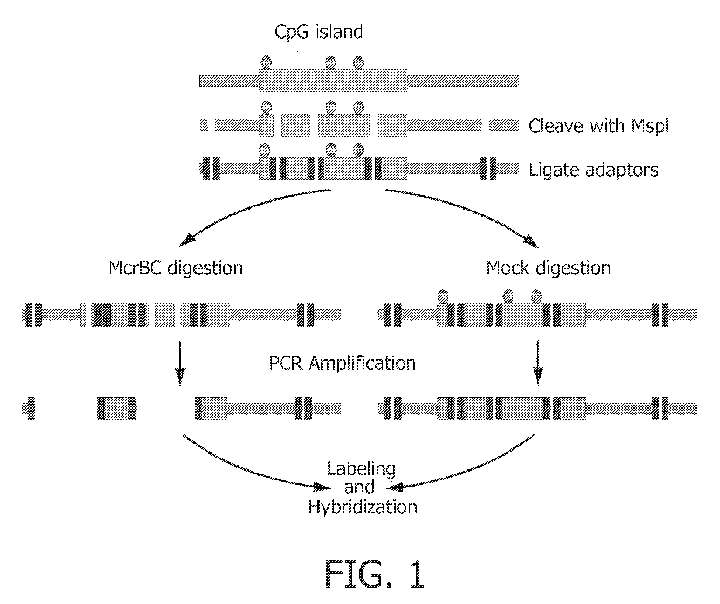

Then, the genomic DNA sample is treated such that cytosine bases which are unmethylated at the 5'-position are converted to uracil, thymine, or another base which does not resemble cytosine in terms of hybridization behavior. This will be understood as 'pretreatment' hereinafter. The treatment of genomic DNA is preferably carried out by using bisulfite (sulfite, disulfite) and subsequent alkaline hydrolysis resulting in a conversion of unmethylated cytosine bases to uracil. If bisulfite solution is used for the reaction, then an nucleophilic addition takes place at the unmethylated cytosines. In addition, a denaturating reagent or solvent as well as a radical interceptor must be present. The converted DNA is then used for the detection of methylated cytosine residues (cf. Fig. 1).

The respective DNA fragments to be analyzed were amplified. Because of statistical and practical considerations, preferably more than ten different fragments having a length of 100 bp - 2000 bp were amplified. The amplification of several DNA segments can be carried out simultaneously in one and the same reaction vessel. Usually, the amplification is carried out by means of a polymerase chain reaction (PCR).

The design of oligonucleotide primers to be used in a PCR is obvious to one skilled in the art. Preferably, the primers do not contain any CpG dinucleotides. The sequence of said primer oligonucleotides are designed to selectively anneal to and amplify only a specific DNA fragment of interest, thereby minimizing the amplification of background or non relevant DNA.

In specific embodiments, at least one primer oligonucleotide is bound to a solid phase during amplification. The different oligonucleotide sequences can be arranged on a plane solid phase in the form of a rectangular or hexagonal lattice, the solid phase surface *inter alia* being composed of silicon, glass, polystyrene, aluminum, steel, iron, copper, nickel, silver, or gold, nitrocellulose or plastics. The fragments obtained may carry a directly or indirectly detectable label. Preferably, the labels employed are fluorescence labels, radiolabels, or detachable molecule fragments having a typical mass which can be detected in a mass spectrometer.

Subsequently, the nucleic acid amplicons are analyzed in order to determine the methylation status of the genomic DNA prior to treatment. Several methods for the methylation status specific analysis of the treated nucleic acids are known, other alternative methods will be obvious to one skilled in the art. For example, the analysis may be carried out during the amplification step. In such scenario, the methylation status of preselected CpG positions within the nucleic acids may be detected by use of methylation specific primer oligonucleotides (cf., e.g., US Patent 6,265,171).

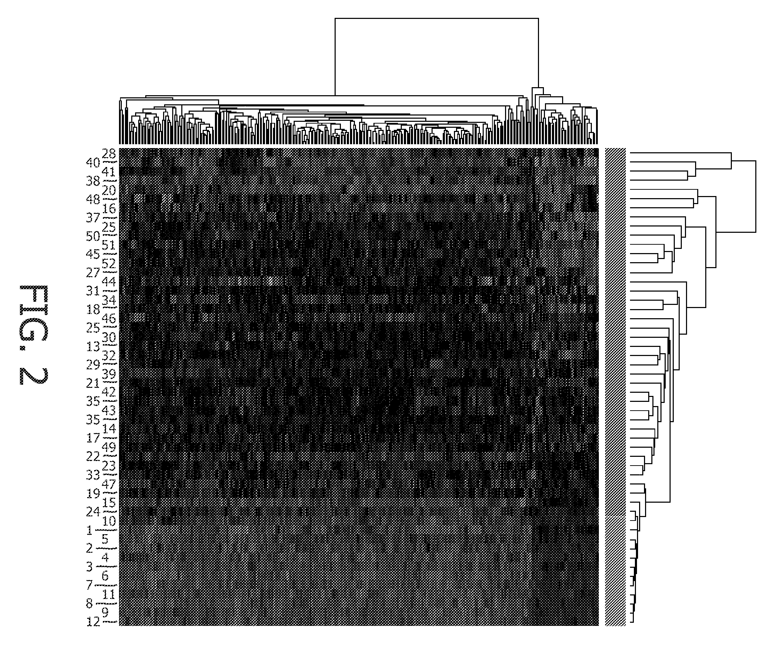

Figure 2 shows an exemplary distribution of clustered samples (columns) vs. methylation loci (rows). The DNA methylation patterns obtained allow for a differentiation between tumors (left part of bar on top) and normal tissue (right part of bar on top).

## 5. Prediction of DNA methylation patterns

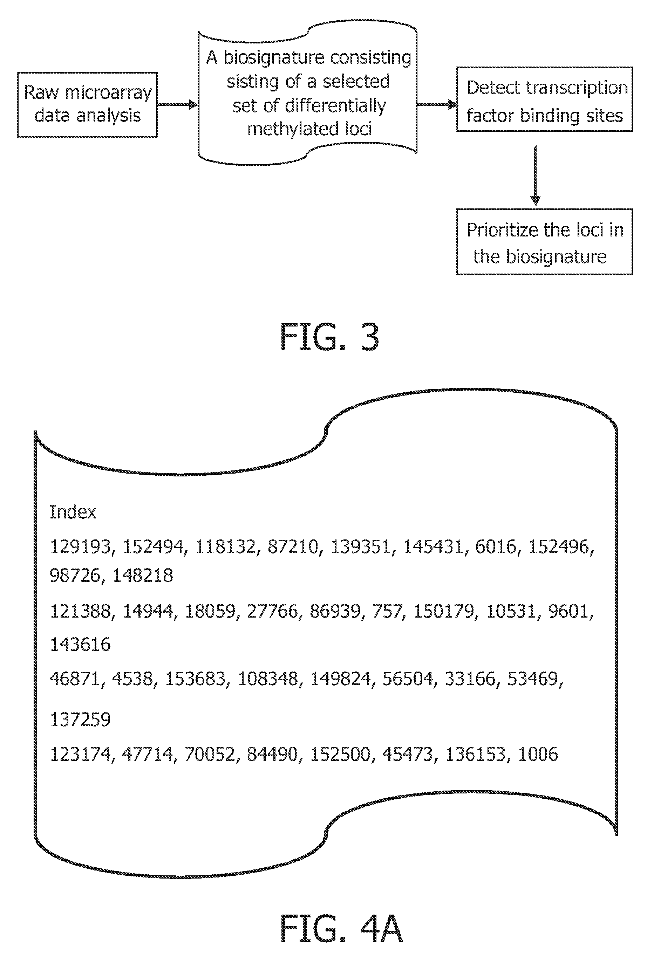

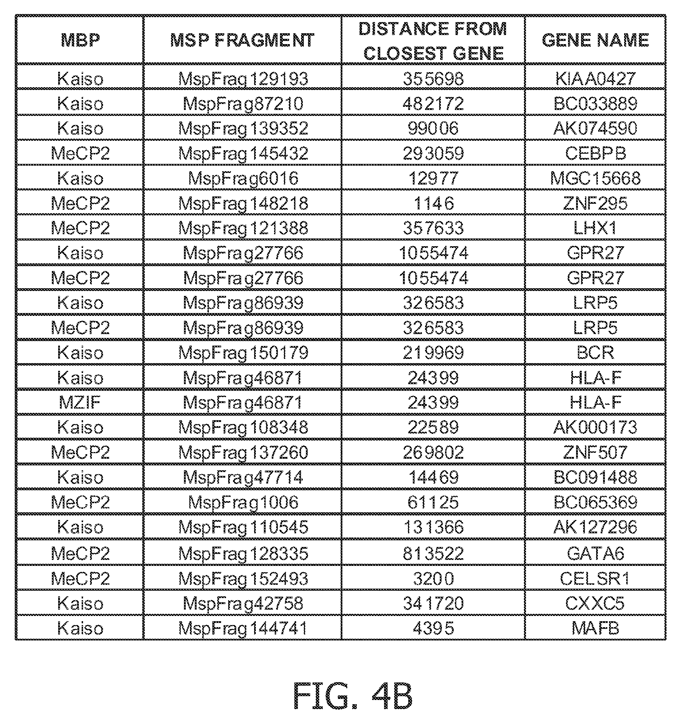

First, a data set of differentially methylated genes (or other loci) has to be generated by using either a low throughput assay such as methylation specific PCR (MSP) or a high throughput method (e.g., MOMA, HELP assay, methyl-DIP or Illumina's methylation bead array technology). A schematic representation of the principle underlying the method according to the invention is depicted in Fig. 3. An exemplary DNA methylation signature/biosignature for distinguishing between different subtypes of breast cancer is shown in Fig. 4.

If a low throughput assay is used such as methylation specific PCR, further information regarding the target sequences are required. Usually, there are no direct hits when DNA segments corresponding to primers used in the assay are aligned with the human genome sequence using the BLAT genome database (UCSC Genome Bioinformatics Group, SantaCruz, CA, USA). Therefore, the primer sequences need to be aligned with the genomic sequence of corresponding genes obtained from human genome. Optionally, instead of a "wet biology experiment" an *in silico* PCR may be performed with the primers obtained after aligning MSP primers with human genome.

A high throughput method such as generates raw data for differential DNA methylation analysis. In some specific embodiments, the two or more matched samples for hybridization may be aggressive *vs.* non-aggressive cancer, or patients who responded to therapy *vs.* those who did not respond to therapy (such as chemotherapy, immunotherapy, or a combination of therapies).

Automatic selection of differential methylation gene signatures: This goal may be accomplished by using traditional statistical (e.g., Student's t-test) or machine learning approaches (e.g., SVM). This step produces one or many candidate genes (or one or more candidate signatures each consisting of a set of genes). The further method steps aim at providing a way for the incorporation of biological knowledge about the epigenetic mechanism of gene silencing via methyl-binding proteins (and/or via other DNA binding factors such as transcription factors).

The one or more candidate genes (optionally within each signature) are separated into two groups:

(a) those comprising a change from the unmethylated to the methylated state; in the context of diagnosing/monitoring a cancerous state such "hypermethylation" is indicative of tumor suppressor gene inactivation (hereinafter, these genes are thus referred to as "T set"); and

(b) those comprising a change from the methylated to the unmethylated state; in the context of diagnosing/monitoring a cancerous state such "hypomethylation" is indicative of oncogene activation (hereinafter, these genes are thus referred to as "O set").

For the subsequent correlation of the methylation level with the presence of DNA binding factor recognition sites (transcription factor binding sits) the following steps are required:

(1) The differentially methylated candidate gene sequences in the T set are used as the foreground set and those of the O set as the background set for finding transcription factor binding sites (TFBSs). If a candidate locus has (i.e. the site of differential methylation) a TFBS, then it is included in the next stage of analysis. If the locus is going to play an epigenetic role related to a tumor suppressor gene, then the transcription factor (TF) cannot bind to this locus due to the methylation, and therefore part of the signaling network is broken.

(2) The differentially methylated candidate gene sequences in the O set are used as the foreground set and those of the T set as the background set for finding TFBSs. If a candidate locus has a TFBS then it is included in the next stage of analysis. It has to be confirmed as well that the respective TF is also not methylated so that there is higher chance that the locus is of functional importance. In such case, both the unmethylated locus and the unmethylated TF are included in the final signature.

(3) For each gene/locus in the T-set, it is further determined whether a consensus binding sequence of a DNA methyl-binding protein (such as MBD1, MBD2, MBD3 and MBD4, Kaiso, and MeCP2), a partiular type of TF, is present. Then, gene networks that are regulated by a single methyl binding protein are derived. A candidate locus having a methyl protein binding site is included in the next stage of analysis.

Next, the differentially methylated candidate genes are evaluated to become part of a methylation signature based on the above-mentioned three steps. A gene/locus needs to participate in at least one of the respective networks/subsets. The pathway information is included from one of the pathway databases (e.g., KEGG pathway database). The remainder of the loci is discarded.

A candidate gene is in the final set if the combined values of differential methylation (M_level), presence of methyl binding protein sites (No_MBS) and presence of transcription factor binding sites (No_TFBS) as expressed in the M_Score formula is higher than a certain threshold:

$$M\_Score = w1 * M\_Level + w2 * No\_MBS + w3 * No\_TFBS$$

This evaluation may confirm existing and new dependencies and gene cascades in gene networks. This would also further elucidate the biomarkers down the cascades that may be used as surrogates for DNA methylation evaluation.

For all these genes/loci a higher confidence is required that the specific locus should be in the final set. In this manner, loci having a higher potential to play an epigenetic role are include. Of course, to confirm a functional role for a methylated site, there should be a functional assay performed: for example, the cells may be treated with a demethylating agent such as 5-azacitidine followed by a gene expression assay to confirm that demethylation of the locus is correlated with regulation of the respective gene.

The invention can be used for candidate biomarker/bio-signature evaluation and selection. Appropriate bio-signatures derived from this method can be used for the classification of patients, screening, diagnosis, prognostication, therapy planning and evaluating recurrence of a disease, e.g., of cancer. In addition, this method can be used as part of an *in silico* analysis pipeline to make hypothesis for epigenetic studies in any organism. In addition to the use of this method for research purposes, the method of the present invention may also be used in a computer-based clinical decision system along with other patient data and clinical parameters.

## Claims

1. Method for correlating a DNA methylation signature with the transcriptional silencing of a candidate gene, the method comprising:

(a) providing a plurality of at least two matched samples, the plurality comprising at least one target sample and at least one reference sample;

(b) selecting one or more candidate genes/loci that exhibit differential DNA methylation in the at least one target sample as compared to the at least one reference sample;

(c) determining the nucleic acid sites comprised in the one or more candidate genes/loci obtained in step (b) that are differentially methylated and subsequently dividing the one or more candidate genes/loci that are differentially methylated in

- a first subset "m" of one or more candidate genes/loci comprising nucleic acid sites which are methylated in the at least one reference sample and unmethylated in the at least one target sample; and
- a second subset "n" of one or more candidate genes/loci comprising nucleic acid sites which are unmethylated in the at least one reference sample and methylated in the at least one target sample; and

(d) determining in the one or more candidate genes/loci obtained in step (b) the presence of recognition sites for DNA binding factors, wherein said DNA binding factors each recognize a nucleic acid site determined in step (c), wherein said step comprises

- determining and selecting the recognition sites for a first subset "M" of one or more DNA binding factors, wherein each member of the subset "M" of DNA binding factors selectively recognizes one or more candidate genes of the subset "m";
- determining and selecting the recognition sites for a second subset "N" of one or more DNA binding factors, wherein each member of the subset "N" of DNA binding factors selectively recognizes one or more candidate genes of the subset "n"; and

(e) determining a DNA methylation signature which is represented by the pattern of the differentially methylated nucleic acid sites obtained in step (c) and by the pattern of DNA binding factor recognition sites obtained in step (d) wherein the DNA methylation signature is indicative for the presence of or the predisposition to develop cancer in the at least one target sample, wherein

- the nucleic acid sites comprised in the one or more candidate genes/loci that are differentially methylated are CpG dinucleotide sites,
- the DNA methylation signature identified for the subset "m" of candidate genes/loci is indicative of the activation of one or more oncogenes, and
- the DNA methylation signature identified for the subset "n" of candidate genes/loci is indicative of the inactivation of one or more tumor suppressor genes..

2. The method of claim 1, wherein differential DNA methylation is determined by means of one or more methods selected form the group of bisulfite sequencing, pyro-sequencing, methylation-sensitive single-strand conformation analysis (MS-SSCA), high resolution melting analysis (HRM), methylation-sensitive single nucleotide primer extension (MS-SnuPE), base-specific cleavage/MALDI-TOF, methylation-specific PCR (MSP), microarray-based methods, and *Msp*I cleavage.

3. The method of claim 1 or 2, wherein the subset "N" of DNA binding factors represents DNA methyl-binding proteins.

4. The method of claim 3, wherein the DNA methyl-binding proteins are selected from the group of MBD1, MBD2, MBD3, MBD4, MIZF, Kaiso, and MeCP2.

5. The method of any one of claims 1 to 4, further comprising one or more repetitions of step (d), wherein each repetition comprises determining in the one or more candidate genes/loci the presence of recognition sites for one or more DNA binding factors that have not been included in the determination of the previous repetitions.

6. The method of any one of claims 1 to 5, wherein the DNA methylation signature identified comprises at least ten candidate genes/loci.

7. The method of any one of claims 1 to 6, for the further use of predicting the therapeutic response to the treatment of the cancer present or predisposed to develop in the at least one target sample.

8. The method of any one of claims 1 to 7, wherein the method is performed *in silico*.

9. Use of a DNA methylation signature as defined in any one of claims 1 to 8 as a biomarker for the classification of patient samples for screening, diagnosing, therapy planning and/or recurrence monitoring of a cancer.

10. Use of the method of any one of claims 1 to 9 as an integral part of a computer-based clinical decision system along with other patient data and clinical parameters.

**Patentansprüche**

1. Verfahren zum Korrelieren einer DNA-Methylierungssignatur mit dem transkriptionellen Stummschalten eines Kandidatengens, wobei das Verfahren Folgendes umfasst:

   (a) Bereitstellen einer Vielzahl von mindestens zwei übereinstimmenden Proben, wobei die Vielzahl mindestens eine Zielprobe und mindestens eine Referenzprobe umfasst:
   (b) Auswählen von einem oder mehreren Kandidatengenen/Loki, die eine unterschiedliche DNA-Methylierung in der mindestens einer Zielprobe im Vergleich zu der mindestens einen Referenzprobe aufweisen;
   (c) Ermitteln der in dem einen oder mehreren Kandidatengenen/Loki, die in Schritt (b) erlangt wurden, enthaltenen Nukleinsäurestellen, die unterschiedlich methyliert sind, und anschließendes Aufteilen der einen oder mehreren Kandidatengene/Loki, die unterschiedlich methyliert sind, in

      - eine erste Untergruppe "m" von einem oder mehreren Kandidatengenen/Loki umfassend Nukleinsäurestellen, die in der mindestens einen Referenzprobe methyliert sind und in der mindestens einen Zielprobe unmethyliert sind; und
      - eine zweite Untergruppe "n" von einem oder mehreren Kandidatengenen/Loki umfassend Nukleinsäurestellen, die in der mindestens einen Referenzprobe unmethyliert sind und in der mindestens einen Zielprobe methyliert sind; und

   (d) Ermitteln der Anwesenheit von Erkennungsstellen für DNA-Bindungsfaktoren in dem einen oder mehreren der in Schritt (b) erlangten Kandidatengenen/Loki, wobei die genannten DNA-Bindungsfaktoren jeweils eine in Schritt (c) ermittelte Nukleinsäurestelle erkennen,
   wobei der genannte Schritt Folgendes umfasst:

      - Ermitteln und Auswählen der Erkennungsstellen für eine erste Untergruppe "M" der einen oder mehreren DNA-Bindungsfaktoren, wobei jedes Mitglied der Untergruppe "M" der DNA-Bindungsfaktoren selektiv ein oder mehrere Kandidatengene der Untergruppe "m" erkennt;
      - Ermitteln und Auswählen der Erkennungsstellen für eine zweite Untergruppe "N" der einen oder mehreren DNA-Bindungsfaktoren, wobei jedes Mitglied der Untergruppe "N" der DNA-Bindungsfaktoren selektiv ein oder mehrere Kandidatengene der Untergruppe "n" erkennt; und

   (e) Ermitteln einer DNA-Methylierungssignatur, die durch das Muster der unterschiedlich methylierten Nukleinsäurestellen, die in Schritt (c) erlangt wurden, und durch das Muster der DNA-Bindungsfaktor-Erkennungsstellen, die in Schritt (d) erlangt wurden, dargestellt wird, wobei die DNA-Methylierungssignatur die Anwesenheit von oder die Prädisposition für die Entwicklung von Krebs in der mindestens einen Zielprobe angibt, wobei

      - die Nukleinsäurestellen, die in dem einen oder mehreren Kandidatengenen/Loki enthalten sind, welche unterschiedlich methyliert sind, CpG-Dinukleotidstellen sind,
      - die für die Untergruppe "m" von Kandidatengenen/Loki identifizierte DNA-Methylierungssignatur die Aktivierung von einem oder mehreren Onkogenen angibt,
      und
      - die für die Untergruppe "n" von Kandidatengenen/Loki identifizierte DNA-Methylierungssignatur die Inaktivierung von einem oder mehreren Tumorsuppressorgenen angibt.

2. Verfahren nach Anspruch 1, wobei die unterschiedliche DNA-Methylierung mithilfe von einem oder mehreren Verfahren ausgewählt aus der Gruppe bestehend aus Bisulfit-Sequenzierung, Pyrosequenzierung, methylierungssensitive Einzelstrang-Konformationsanayse (MS-SSCA), hochauflösende Schmelzanalyse (HRM), methylierungssensitive Einzel-Nukleotid-Primer-Extension (MS-SnuPE), basenspezifische Spaltung/MALDI-TOF, methylierungssensitive PCR (MSP), mikroarray-basierende Verfahren und MspI- Spaltung ermittelt wird.

3. Verfahren nach Anspruch 1 oder 2, wobei die Untergruppe "N" von DNA-Bindungsfaktoren DNA-Methyl-Bindungsproteine darstellt.

**4.** Verfahren nach Anspruch 3, wobei die DNA-Methyl-Bindungsproteine ausgewählt werden aus der Gruppe bestehend aus MBD1, MBD2, MBD3, MBD4, MIZF, Kaiso und MeCP2.

**5.** Verfahren nach einem der Ansprüche 1 bis 4, weiterhin umfassend eine oder mehrere Wiederholungen von Schritt (d), wobei jede Wiederholung das Ermitteln der Anwesenheit von Erkennungsstellen für einen oder mehrere DNA-Bindungsfaktoren in dem einen oder mehreren Kandidatengenen/Loki umfasst, die nicht in der Ermittlung der vorhergehenden Wiederholungen enthalten waren.

**6.** Verfahren nach einem der Ansprüche 1 bis 5, wobei die identifizierte DNA-Methylierungssignatur mindestens zehn Kandidatengene/Loki umfasst.

**7.** Verfahren nach einem der Ansprüche 1 bis 6 für die weitere Verwendung zur Vorhersage des therapeutischen Ansprechens auf die Behandlung von in der mindestens einen Zielprobe vorhandenen oder zur Entwicklung prädisponierten Krebszellen.

**8.** Verfahren nach einem der Ansprüche 1 bis 7, wobei das Verfahren *in silico* durchgeführt wird.

**9.** Verwendung einer DNA-Methylierungssignatur nach einem der Ansprüche 1 bis 8 als Biomarker für die Klassifizierung von Patientenproben für das Screening, für die Diagnosestellung, für die Therapieplanung und/oder die Überwachung des Wiederauftretens einer Krebserkrankung.

**10.** Verwendung des Verfahrens nach einem der Ansprüche 1 bis 9 als einen integralen Bestandteil eines computerbasierten Entscheidungssystems zusammen mit anderen Patientendaten und klinischen Parametern.

**Revendications**

**1.** Procédé de corrélation d'une signature de méthylation d'ADN avec le silençage transcriptionnel d'un gène candidat, le procédé comprenant :

(a) la fourniture d'une pluralité d'au moins deux échantillons appariés, la pluralité comprenant au moins un échantillon cible et au moins un échantillon de référence ;
(b) la sélection d'un ou plusieurs gènes/loci candidats qui présentent une méthylation différentielle d'ADN dans ledit au moins un échantillon cible par comparaison avec ledit au moins un échantillon de référence ;
(c) la détermination des sites d'acide nucléique compris dans le ou les gènes/loci candidats obtenus à l'étape (b) qui sont méthylés différentiellement et la division ultérieure du ou des gènes/loci candidats qui sont méthylés différentiellement en

- un premier sous-ensemble « m » d'un ou plusieurs gènes/loci candidats comprenant des sites d'acide nucléique qui sont méthylés dans ledit au moins un échantillon de référence et non méthylés dans ledit au moins un échantillon cible ; et
- un second sous-ensemble « n » d'un ou plusieurs gènes/loci candidats comprenant des sites d'acide nucléique qui sont non méthylés dans ledit au moins un échantillon de référence et méthylés dans ledit au moins un échantillon cible ; et

(d) la détermination dans le ou les gènes/loci candidats obtenus à l'étape (b) de la présence de sites de reconnaissance pour des facteurs de liaison d'ADN, dans lequel lesdits facteurs de liaison d'ADN reconnaissent chacun un site d'acide nucléique déterminé à l'étape (c), dans lequel ladite étape comprend

- la détermination et la sélection des sites de reconnaissance pour un premier sous-ensemble « M » d'un ou plusieurs facteurs de liaison d'ADN, dans lequel chaque élément du sous-ensemble « M » de facteurs de liaison d'ADN reconnaît sélectivement un ou plusieurs gènes candidats du sous-ensemble « m » ;
- la détermination et la sélection des sites de reconnaissance pour un second sous-ensemble « N » d'un ou plusieurs facteurs de liaison d'ADN, dans lequel chaque élément du sous-ensemble « N » de facteurs de liaison d'ADN reconnaît sélectivement un ou plusieurs gènes candidats du sous-ensemble « n » ; et

(e) la détermination d'une signature de méthylation d'ADN qui est représentée par le motif des sites d'acide nucléique méthylés différentiellement obtenu à l'étape (c) et par le motif de sites de reconnaissance de facteur de liaison d'ADN obtenu à l'étape (d) dans lequel la signature de méthylation d'ADN est indicative de la présence ou de la prédisposition à développer un cancer dans ledit au moins un échantillon cible, dans lequel

- les sites d'acide nucléique compris dans le ou les gènes/loci candidats qui sont méthylés différentiellement sont des sites de dinucléotide CpG,
- la signature de méthylation d'ADN identifiée pour le sous-ensemble « m » de gènes/loci candidats est indicative de l'activation d'un ou plusieurs oncogènes,
et
- la signature de méthylation d'ADN identifiée pour le sous-ensemble « n » de gènes/loci candidats est indicative de l'inactivation d'un ou plusieurs gènes suppresseurs de tumeur.

2. Procédé selon la revendication 1, dans lequel la méthylation différentielle d'ADN est déterminée au moyen d'un ou plusieurs procédés sélectionnés dans le groupe de séquençage au bisulfite, pyro-séquençage, analyse de conformation de simple brin sensible à la méthylation (MS-SSCA), analyse de fusion à haute résolution (HRM), extension d'amorce de nucléotide unique sensible à la méthylation (MS-SnuPE), clivage spécifique à la base/MALDI-TOF, PCR spécifique à la méthylation PCR (MSP), procédés à base de micromatrice, et clivage Mspl.

3. Procédé selon la revendication 1 ou 2, dans lequel le sous-ensemble « N » de facteurs de liaison d'ADN représente des protéines de liaison de méthyle d'ADN.

4. Procédé selon la revendication 3, dans lequel les protéines de liaison de méthyle d'ADN sont choisies parmi le groupe de MBD1, MBD2, MBD3, MBD4, MIZF, Kaiso et MeCP2.

5. Procédé selon l'une quelconque des revendications 1 à 4, comprenant en outre une ou plusieurs répétitions de l'étape (d), dans lequel chaque répétition comprend la détermination dans le ou les gènes/loci candidats de la présence de sites de reconnaissance pour un ou plusieurs facteurs de liaison d'ADN qui n'ont pas été inclus dans la détermination des précédentes répétitions.

6. Procédé selon l'une quelconque des revendications 1 à 5, dans lequel la signature de méthylation d'ADN identifiée comprend au moins dix gènes/loci candidats.

7. Procédé selon l'une quelconque des revendications 1 à 6, pour l'utilisation supplémentaire de prévision de la réponse thérapeutique au traitement du cancer présent ou prédisposé à se développer dans ledit au moins un échantillon cible.

8. Procédé selon l'une quelconque des revendications 1 à 7, où le procédé est effectué in silico.

9. Utilisation d'une signature de méthylation d'ADN telle que définie dans l'une quelconque des revendications 1 à 8 en tant que biomarqueur pour la classification d'échantillons de patient pour criblage, diagnostic, planification de thérapie et/ou surveillance de récidive d'un cancer.

10. Utilisation du procédé selon l'une quelconque des revendications 1 à 9 en tant que partie intégrante d'un système de décision clinique par ordinateur en même temps que d'autres données de patient et d'autres paramètres cliniques.

FIG. 1

FIG. 2

Raw microarray data analysis → A biosignature consisting sisting of a selected set of differentially methylated loci → Detect transcription factor binding sites → Prioritize the loci in the biosignature

## FIG. 3

Index

129193, 152494, 118132, 87210, 139351, 145431, 6016, 152496, 98726, 148218

121388, 14944, 18059, 27766, 86939, 757, 150179, 10531, 9601, 143616

46871, 4538, 153683, 108348, 149824, 56504, 33166, 53469, 137259

123174, 47714, 70052, 84490, 152500, 45473, 136153, 1006

## FIG. 4A

| MBP | MSP FRAGMENT | DISTANCE FROM CLOSEST GENE | GENE NAME |
|---|---|---|---|
| Kaiso | MspFrag129193 | 355698 | KIAA0427 |
| Kaiso | MspFrag87210 | 482172 | BC033889 |
| Kaiso | MspFrag139352 | 99006 | AK074590 |
| MeCP2 | MspFrag145432 | 293059 | CEBPB |
| Kaiso | MspFrag6016 | 12977 | MGC15668 |
| MeCP2 | MspFrag148218 | 1146 | ZNF295 |
| MeCP2 | MspFrag121388 | 357633 | LHX1 |
| Kaiso | MspFrag27766 | 1055474 | GPR27 |
| MeCP2 | MspFrag27766 | 1055474 | GPR27 |
| Kaiso | MspFrag86939 | 326583 | LRP5 |
| MeCP2 | MspFrag86939 | 326583 | LRP5 |
| Kaiso | MspFrag150179 | 219969 | BCR |
| Kaiso | MspFrag46871 | 24399 | HLA-F |
| MZIF | MspFrag46871 | 24399 | HLA-F |
| Kaiso | MspFrag108348 | 22589 | AK000173 |
| MeCP2 | MspFrag137260 | 269802 | ZNF507 |
| Kaiso | MspFrag47714 | 14469 | BC091488 |
| MeCP2 | MspFrag1006 | 61125 | BC065369 |
| Kaiso | MspFrag110545 | 131366 | AK127296 |
| MeCP2 | MspFrag128335 | 813522 | GATA6 |
| MeCP2 | MspFrag152493 | 3200 | CELSR1 |
| Kaiso | MspFrag42758 | 341720 | CXXC5 |
| Kaiso | MspFrag144741 | 4395 | MAFB |

# FIG. 4B

## REFERENCES CITED IN THE DESCRIPTION

**Patent documents cited in the description**

- US 20060292564 A1
- US 6265171 B

**Non-patent literature cited in the description**

- **WILSON, G.G. ; MURRAY, N.E.** *Annu. Rev. Genet.,* 1991, vol. 25, 585-627
- **LI, E.** *Nat. Rev. Genet.,* 2002, vol. 3, 662-673
- **ROBERTSON, K.D. ; WOLFFE, A.P.** *Nat. Rev. Genet.,* 2000, vol. 1, 11-19
- **BIRD, A.P.** *Genes Dev.,* 2002, vol. 16, 6-21
- **REIN, T. et al.** *Nucleic Acids Res.,* 1998, vol. 26, 2255-2264
- **KLOSE, R.J. ; BIRD, A.P.** *Trends Biochem. Sci.,* 2006, vol. 31, 89-97
- **WINGENDER, E. et al.** *Nucleic Acids Res.,* 1996, vol. 24, 21-25
- **BIRD, A.P. ; WOLFFE, A.P.** *Cell,* 1999, vol. 99, 451-454
- **WADE, P.A.** *BioEssays,* 2001, vol. 23, 1131-1137
- **HENDRICH, B. ; TWEEDIE, S.** *Trends Genet,* 2003, vol. 19, 269-277
- **GARDINER-GARDEN, M. ; FROMMER, M.** *J. Mol. Biol.,* 1987, vol. 196, 261-82
- **LUCITO, R. et al.** *Genome Res.,* 2003, vol. 13, 2291-2305
- **BOLSTAD, B.M. et al.** *Bioinformatics,* 2003, vol. 19, 185-193
- **BENJAMINI, Y. ; HOCHBERG, Y.** False Discovery Rate. *J. Roy. Stat. Soc.,* 1995, vol. 57, 289-300
- **LIN, C.J.** *Neural Computation,* 2001, vol. 13, 307-317